# EUROPEAN PATENT SPECIFICATION

(11) **EP 1 097 681 B1**
(45) Date of publication and mention of the grant of the patent: **19.05.2004**
(21) Application number: 99928270.0
(22) Date of filing: 11.06.1999
(51) Int. Cl.: A61N 5/067, A61F 9/008

(54) **DEVICE FOR LASER THERAPY IN OPHTHALMOLOGY AND VARIANTS**
VORRICHTUNG FÜR DIE LASERTHERAPIE IN DER OPHTALMOLOGIE UND VARIANTEN
DISPOSITIF DE THERAPIE LASER EN OPHTALMOLOGIE ET VARIANTES

(30) Priority: 11.06.1998 RU 98110987
(43) Date of publication of application: 09.05.2001
(73) Proprietor: Utar International Ltd., Bridgetown (BB)
(72) Inventor: ANIKINA, Elena Borisovna, Moscow, 117513 (RU); TURKOV, Jury Grigorievich, Moscow, 121471 (RU)
(74) Representative: W.P. THOMPSON & CO.
(86) International application number: PCT/RU1999/000196
(87) International publication number: WO 1999/063916

(56) References cited:
- WO-A-95/02356
- DE-A- 4 442 071
- RU-C1- 2 092 140
- RU-C1- 2 102 951
- KATSNELSON L.A. et al., Primenenie Nizkoenergeticheskogo Infrakrasnogo Lazernogo Izluchenya Dlya Uluchshenya Krovosnabzhenya i Povyshenya Funktsii Zrenya u Bolnykh s Zabolevanyami Setchatki, M., MNIIGB im. Gelmgoltsa, 1996, pages 3-5.

## Description

### Field of the Invention

The invention relates to medical equipment, namely to the devices for laser therapy in ophthalmology.

### Background of the Invention

Therapeutical effect of the action of laser radiation is usually related to the improvement of the blood circulation in the vascular system of the eye, as well as to the direct bio-stimulation of cells. This effect is expressed, in particular, in raising a vision acuity, removing a spasm of adaptation tension, which decreases the risk of myopia progressing and weariness during the visual work at a close distance [1,2].

A stimulating influence of a trans-scleral laser action is strengthened when using the radiation of a near infra-red range of the spectrum, having a deeper depth of penetration into a tissue compared to visible radiation [1]. At the same time during conducting laser therapy with infra-red radiation, problems arise (due to its invisibility) related to guiding the radiation to the selected areas of the eye. This is important not only from the point of view of the effectiveness of therapeutical procedures, but from the point of view of their safety as well: permissible levels of irradiating tissues of eye muscles and retina are different for the orders of magnitude. The research with the participation of one of the authors has shown the possibility of the solution of these problems while keeping the sight of a patient in certain reference directions during laser therapy session.

These problems were partially solved in the prior art device for laser therapy in ophthalmology [3] representing the preamble of claims 1 and 4. This device comprises two parallel optical channels (optical units) fixed in the spectacles (glasses) rims, each of which has a lasing emitter for trans-scleral action by laser radiation upon two areas of prelimbic region of the eye during a specified period of time, a source of light and forming optics, as well as a power supply and control unit, equipped with a timer for setting a time of laser action. The source of light (a light diode) in each optical unit forms a luminous aiming mark at which a sight of a patient is fixed, and assigns a reference direction by that. The axes of a source of light and of forming optics coincide during this. In addition to that this device contains an adjustment mechanism, on which a lasing emitter, source of light and forming optics are installed with a possibility of displacement in the direction transversal to its axis. Before the beginning of work a patient tries to bring the..... visual images of two sources of light observed by each eye into one. In this case the axes of optical units match with the visual axes of the eyes, and, correspondingly, the distance between parallel axes of these units match with the interpupillary distance of the patient. This is used in the prior art for providing simultaneous action of laser radiation upon the selected areas of the prelimbic region of each of the eyes and for decreasing a labour consuming nature and duration of the laser therapy procedure. When visual images of light are put together into one, the device is considered prepared for work, which serves as a basis for switching on lasing emitters of both units. Luminous marks from sources of light are synchronously glittering during the work of lasing emitters, specified by a time, keeping the sight of the patient in reference directions and guaranteeing exact laser radiation hitting the selected regions of each eye - in the field of ciliary muscle projection upon sclera at 3 and 9 hours for affecting the ciliary body.

However a long operation of the device according to the prior art [3] has shown that it has limited possibilities. The technical solution used in it, although provides for necessary laser therapy safety, is not always acceptable due to superfluous visual tension appearing while keeping the sight of the patient in specified reference directions, which causes discomfort, and undesirable side effects in some categories of patients. It is specially related to patients having a high degree myopia, as well as to small children in a general case. These patients suffer visual overstrain, and in a number of cases cannot match visual images of glittering luminous marks. It is in particular expressed in involuntary wish to "assist" to himself by way of changing the mutual setting of optical units and locating them under more convenient angle with respect to each other which causes deformation and impairing the instrument design (during a laser therapy session a patient holds the body of optical units with two hands). Such a visual overstrain counteracts to laser therapy and not only decreases the effectiveness of treatments, but also can increase the risk of undesirable side effects. This is explained by the presence of interconnection between accommodation and convergence, which becomes especially strict in case of the eyes mismatch. In this case for each diopter of accommodation tension there is a certain value of visual axes convergence, called the relation of accommodation convergence to convergence (AC/C). This value is different with different patients. The relation AC/C has the biggest values in the patients with myopia [4].

The restriction of possibilities of the prior art, described above, are stipulated by the fact that the technical solution used in it does not create adequate conditions for conducting laser therapy, corresponding to the above individual peculiarities of the patients, because it restricts the possibility of patients sight fixation by only those reference directions which are parallel to each other. This follows from the requirement of parallelism of optical units location in this device and of matching the axes of the corresponding sources of light and forming optics in these units.

### Summary of the Invention

According to the present invention, there is provided a device for laser therapy according to claim 1. There is further provided a device for laser therapy according to claim 4.

From the above follows the necessity of such a solution of the task of aiming radiation at the selected zones (sections of prelimbic area) of the eye during laser therapy by infrared radiation, which would provide for fixation of the patients sight in such reference directions, which match with his individual peculiarities. Positive solution of this task will lead to raising the effectiveness of laser therapy, lowering patient's discomfort in the process of conducting it, as well as will lead to the absence or essential reduction of the above undesirable manifestation at the expense of decreasing visual tension while keeping the patient's sight in these reference directions.

The task put is solved by the device for laser therapy in ophthalmology comprising two optical units, each of which having one or several lasing emitters with the forming optics for trans-scleral action by laser radiation upon one or several regions of the prelimbic area of the eye, as well as comprising the source of light for patient's sight fixation in a reference direction, and providing by that the indicated action upon the said areas of the eye, meanwhile the optical units are installed on the adjustment mechanism with the possibility of a displacement of at least one of them in the direction transversal to its axis for matching their position with the position of visual axes of the patient, while according to the invention, the said optical units are installed at an angle to each other, at which reference directions, specified in these optical units with a help of said sources of light, form an angle between themselves in an angular interval, compatible with the value of convergence of visual axes of the patient during the observation with two eyes.

The essence of the invention is based upon the idea of the authors of coordinating with individual peculiarities of each patient not only of a distance between reference directions in which his sight is being fixed, but also of an angle between them in accordance with the value of visual axes convergence during the observation with both the eyes.

Coordination of the distance between reference directions with the interpupillary distance is necessary for providing a simultaneous action by laser radiation upon the selected sections of the prelimbic area of each eye. It is executed by the patient similarly to the prototype by combining visual images of two sources of light observed by each eye into one (that is their matching) with a help of an adjustment mechanism.

Matching with the value of visual axes convergence is necessary in order to create more comfortable conditions for the above matching of appearing visual images, when for such matching and keeping it during the whole therapy session only minimum visual work and, correspondingly, minimum tension of the eyes are required from the patient. For the implementation of such conditions the patient is either guided by the instructions of a doctor - ophthalmologist, or makes the selection himself, conducting matching of observed images of sources of light for a number of devices in which optical units are installed at different angles to each other. It is expedient for facilitating this selection for different groups of patients that in each such device an angle between such reference directions would belong to one of sequential angular intervals embracing in combination the specified angular range. The patient judges about the correctness of the angular interval selection by his own feelings" according to the minimum of tension when matching the above visual images of the sources of light.

Such selection can be also made with a help of one universal device, if at least one of its optical units is installed on the adjustment mechanism with the possibility of rotating in an azimuthal plane and fixation in the selected position. In this case for the selection of the most comfortable (requiring minimum tensions of accommodation apparatus) angular interval between reference directions of the sight, specified in each optical unit, the patient changes an angle between the optical units with a help of corresponding rotation means and after finding it, fixes the angular position if optical units with a help of a suitable lock. The advantage of such a universal device is in its greater functional possibilities, as before each session of laser therapy a patient can control and change the angle between the optical units, each time creating the most comfortable conditions for himself during conducting this session.

The practice of using this device with such a binocular optical scheme has shown that for the majority of patients with progressing myopia, especially for persons of junior age, minimum visual tension is reached when reference directions of a sight, specified by sources of light in each of optical units, are not parallel to each other, but form a converging angle in the direction of the sight. The minimum value of this angle, at which a substantial effect is already reached in decreasing a visual tension stipulated by the necessity of matching two closely located light marks observed by each eye, is different for different patients. However, as it follows from the available clinical experience, it should for certain exceed 0.01 radian for a rather wide contingent of patient with different pathologies of the accommodation apparatus of the eye.

The maximum value of reference directions convergence angle in two optical units can be evaluated on the data given in work [4] about the relation between accommodation and convergence. In different types of eye refraction (hypermetropia, emmetropia, myopia) the relation between the degree of accommodation and convergence, corresponding to the work zone, are changes within very wide limits. The value corresponding to the angle of convergence of visual axes of both the eyes while looking at an object located at the same distance from the eye, as the sources of light used in the device, can be taken as an evaluating criterion for the upper limit of reference directions convergence angle in two optical units of the proposed device. When the value of the interpupillary distance is 60 mm and the distance to the source of light is 15 cm, the value of reference directions convergence is about 0.4 radian. That is why the maximum value of reference directions convergence can be about 0.5 radian.

The value of angular intervals and their number within specified angular range (for example, the above range from 0.01 to 0.5 radian) can be set based on the experience of work. Even more so, they can be different while working with different groups of patients. In particular, it might be intervals of the same width (for example, five intervals having 0.1 radian each).

It is necessary to bear in mind while selecting the value of an angular interval that a permissible minimum value of angle of reference directions convergence can vary for each specific patient depending on the status of his accommodation apparatus of the eyes. This is confirmed, in particular, by the circumstance that during conducting a course of laser therapy bringing about the raise of ciliary muscle function with a help of device [3], patients' discomfort and the above involuntary wish to deform the device weaken.

It should be pointed out that for the patients having some types of squint, binocular devices can be preferable for conducting laser therapy, in which the above reference directions of a sight form an angle, diverging in the direction of the sight. In this case the range of possible changes of an angle between reference directions of the sight in accordance with the divergence value (that is negative convergence) of visual axes during observation by both eyes can be also established in this case.

It was meant until now in the description of the essence of the invention that the above source of light, assigning a reference direction of the sight in the optical unit occupies a certain fixed position in it: is located on its optical axes or is displaced in the direction, transversal to it, forming a certain angle with it. For example, sources of light can be symmetrically located with respect to a bisectrix of the angle between optical axes of these units. In each such case setting the angle between reference directions in accordance with the individual peculiarities of a patient is performed by way of setting a corresponding angle between optical units. By that, each lasing emitter (for example, a semiconductor laser) is set in a corresponding optical unit into position, at which the beam of its radiation, formed by a corresponding forming optics (for example, lens) is directed at necessary angle to the reference direction set in this unit, so that it hits the section of the prelimbic area of the eye (for example, at 3 or 9 hours) and forms a spot of a specified size (for example, with the diameter of 2-3 mm) in it. The calculation of this angle is made based on the value of transversal displacement of the centre of such spot with respect to the visual axis of the eye and the distance to a lasing emitter. In the case when one lasing emitter is used for irradiating several sections of the eye, the forming optics of this emitter includes a beam splitter (for example, fibreoptical) and lenses, the axes of which are oriented at corresponding angles to the above reference direction. It is clear in this case that neither a number of simultaneously irradiated zones of each eye, nor their position, as well as the number of lasing emitters used, and a corresponding specific design of the forming optics do not restrict the essence of the invention, consisting of the coordination of the angle between the above reference directions with the value of the patient's visual axes convergence while observing with both eyes.

It should be pointed out that such coordination can be conducted in a different way: by setting a corresponding position of the source of light with a fixed angle between optical units. It essentially defines a different variant of the idea of the invention embodiment, in which the task set is solved by the fact that the device for laser therapy in ophthalmology, comprising two optical units, in each of which one or several lasing emitters are set with the forming optics for trans-scleral action by laser radiation upon one or several sections of the prelimbic area of the eye, as well as a source of light for fixing patient's sight in a reference direction and providing by that the indicated action upon the above sections of the eye, while optical units are installed on the adjustment mechanism with the possibility of the displacement of at least one of them in the direction, transversal to its axis for coordinating their position with the position of visual axes of the patient, according to the invention, an the source of light of at least of one of optical units is displaced in the transversal direction to its optical axis for the distance, at which reference directions, set in these optical units with a help of the above sources of light, form an angle between themselves in an angular interval, compatible with the value of patient's visual axes convergence while observing with both eyes.

In this variant the position of optical units themselves is not essential, if in this case the angle between the above reference directions falls into the above angular interval. In particular, the optical units can be set parallelly or at a certain angle to each other (for example, 0.1 radian). The considerations expressed before in respect of the value and the number of angular intervals meant for different groups of patients, the size of the angular range covered by them, the number of simultaneously irradiated zones of each eye and their position, the number of lasing emitters, as well as a specific design of forming optics for different variants of the use of lasing emitters are fully applicable to this variant.

The peculiarity of this variant is a different form of implementation of the change of the angle between said reference directions of the sight;
the indicated source of light and one or several lasing emitters with the forming optics of at least one of the optical units are installed with a possibility of joint movement in the transversal direction to its optical axis and fixation in a selected position.

It is clear that in such an optical unit one or several lasing emitters should be located on the same means of displacement with a source of light (for example on one platform, moving along a guide, transversal to the axis of the optical unit), so that during their joint movement their mutual position could not change, guaranteeing laser radiation hitting specified zones of the eye.

The analysis of the essence of the invention and of its variants conducted confirms the substantiation of the selection of common essential features, describing the applied for device for laser therapy in ophthalmology, and the presence of distinguishing features among them testifies to the correspondence of the applied for invention to the conditions of patentability for novelty.

By that, it follows from the analysis of the state of the art that the solutions of the problem of laser radiation setting at the areas of the eye subject to the action proposed by the authors, allowing to raise the efficiency of laser therapy, decrease the discomfort in its process, as well as to reduce the risk of undesirable side-effects, were not used in this and in related fields of the art. This allows to make a conclusion that the applied for invention corresponds to patentability conditions according to the inventive step.

### Brief Description of the Drawings

The essence of the invention described above is explained below with a specific embodiment.

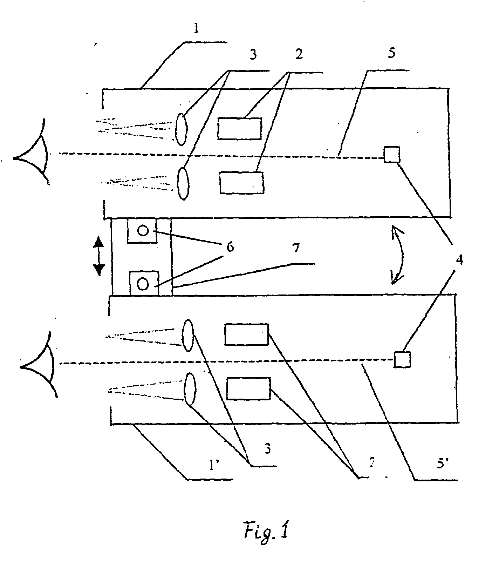

Fig. 1 shows the scheme of one of the variants of the device for laser therapy in ophthalmology.

### Variants of the Invention Embodiment

The device comprises two optical units 1 and 1', in each of which two lasing emitters 2 (semiconductor lasers) are installed, each of which is equipped with the forming optics 3 (in the form of a lens, installed on the axis of this emitter) for trans-scleral action by laser radiation upon a corresponding section (at 3 or 9 hours, is not shown in fig. 1). Besides in each optical unit 1 (1') in its axis a light diode source of light 4 is installed, serving for the formation in the field of vision of a patient of a light mark for fixing the sight in a reference direction 5 (5') during the process of a laser therapy session. By that, lasing emitters are installed in an azimuthal plane, passing through reference directions 5 and 5', symmetrically with respect to a corresponding direction 5 (5') and their optical axis (are not shown in fig. 1) form an angle of an order of 0. 1 radian with this direction for providing by this the above action upon said sections of the eye. For a corresponding change of the angle between reference directions 5 and 5', optical units 1 and 1' are installed on platforms 6 with the possibility of turning around corresponding axes (not shown in fig. 1) in the azimuthal plane and fixation in the selected position. In their turn platforms 6 are installed on guide 7 with a possibility of a longitudinal displacement along it in the direction transversal to the axis of optical unit 1) for coordinating the position of optical units 1 and 1' with the position of visual axes of a patient and fixing units in the selected position. Platforms 6, installed on guide 7, form an adjustment mechanism of the applied for device. The composition of the device also includes, similarly to the prior art, a power supply and control unit (not shown in fig. 1) connected to the sources of light 4 and lasing emitters 2 of optical units 1 and 1', and are equipped with a timer.

The applied for device works in the following way. Before a laser therapy session, optical units 1 and 1' are installed into the initial position: at a minimum angle to each other (for example, 0.01 radian in an absolute value). The same angle is also formed by reference directions 5 and 5' in the azimuthal plane, as for this example of the applied for device embodiment, sources of light 4 are installed in optical axes of units I and 1'. A doctor, with a help of a suitable displacement means (for example, a screw gear) displaces one or both platforms 6 along guide 7 with respect to each other until a distance between optical units 1 and 1' is not set in accordance to an interpupillary distance of the patient which was measured beforehand. Matching of visual images of two sources 4 observed by the patient into one should be provided in this case. If this does not occur at the mutual position of optical units 1 and 1' set or such matching requires tensions of accommodation apparatus of the eyes, then the patient, with a help of suitable rotation means (for example, worm and worm gear) - is not shown in fig. 1) is gradually changing the angle between optical units I and 1'(or, which is equivalent to it, between reference directions 5 and 5') until the matching of visual images of two sources of light 4 is reached, which testifies to the achievement of an angular interval between reference directions 5 and 5', compatible with the value of convergence of patient's visual axes while observing with both eyes. After that optical units 1 and 1' are fixed in the selected position with a help of a suitable fixing device.

During the whole procedure of the device tuning which was described, light diode sources 4 are continuously lit. During this procedure aiming of radiation of corresponding lasing emitters 2 to the sections of prelimbic area (at 3 and 9 hours) is provided. After that with a help of a power supply and control unit laser emitters 2 are switched on for the time, set by the timer. Sources 4 are transferred into a glittering mode for the same period of time for facilitating fixation of patient's sight in reference directions 5 and 5'. After the time set passes, power supply and control unit automatically switches lasing emitters 2 of both optical units 1 and 1' off, informing the patient about it by transferring sources 4 into the mode of continuous lightening and by a sound signal. Upon the end of the session, units are installed into the initial angular position and the device is ready for the operation with the next patient. As during the implementation of the described variant of the applied for device embodiment known elements the production of which is mastered by the industry are used, then it is possible to conclude that it satisfies the conditions of patentability according to industrial applicability.

At the same time the above variant of the device implementation cannot be considered as the restriction of the applied for device. It is only an illustration allowing to better understand its essence which is most completely described in the invention claims.

An example of a specific application of the device according to the invention is offered.

Patient A., 10 years old. Diagnosis - progressing myopia of a weal degree. The acuity of vision of both eye without correction is equal to 0.1, with sph - 3.0 diopters it is 0.9. The patient had a course of laser therapy during 10 days (10 sessions) with a help of a device for laser therapy in its binocular version. The action was applied to a prelimbic area of the eye at 3 and 9 hours by infrared laser radiation with a wave length of 1.3 micron. An aggregate dose of ciliary muscle radiation in each session was about 0.2 J/cm² for each eye. As a result of treatment a reserve of relative accommodation was increased up to -2.5 Diopters. The acuity of vision of both eyes without correction became equal to 0.2, with -2.5 Diopters sph it constituted 1.0. An angle between reference directions of about 0.09 radian had to be installed for the patient for matching visual images of the source of light in two optical units. By that, the treatment session was conducted without a visual tension in a comfortable state of the patient.

The example given confirms the possibility of the implementation of the applied for invention and of raising the effectiveness of laser therapy of eye accommodation apparatus pathologies with its help, decreasing a visual tension and discomfort of the patient during it.

### Industrial applicability

The invention can be used while conducting a stimulation of ciliary muscle for raising effectiveness of accommodation apparatus pathologies therapy, decreasing visual fatigue and discomfort in the process of conducting it, as well as for reducing a risk of undesirable side effects by decreasing a visual tension during keeping a patient's sight in such reference directions, which are compatible with the position and convergence of visual axes of the patient's eye during the observation with two eyes.

### LITERATURE

1. E.B. Anikina, E.I. Shapiro, N.B Baryshnikov, L.S. Orbachevsky, T.A. Pikus. Laser Infrared Therapeutical Device for Treatment of Disorders of Accommodation Ability of the Eye. Thesis of the reports at the 8-th Conference "Laser Optics" and 15-th International Conference on Coherent and Non-Linear Optics, S-P, 1995.
2. V.E. Avetisov, E.B. Anikina, E.V. Akhmedzhanova. The use of helium-neon laser in functional research of the eye and in pleoptical treatment of amblyopia and nystagmus. Methodical recommendations of the Ministry of health of the RSFSR, Moscow Scientific Research Institute of eye Diseases named after Helmgolts, M., 1990, 14 pages
3. M.G. Vasiliev, E.B. Anikina, L.S. Orbachevsky. Device for Laser Therapy in Ophthalmology. RF Patent No. 2092140 of 10.10. 1997 with the priority of 14.10. 1992.
4. E.S. Avetisov. Myopia. Medicine, 1986, pages 19, 61-63.

## Claims

1. A device for laser therapy in ophthalmology, comprising two optical units each having one or several lasing emitters with forming optics for trans-scleral action by laser radiation upon one or several zones of a prelimbic area of the eye, and a source of light for patient's sight fixation specifying a reference direction, in which the forming optics provides for laser radiation hitting said zones of the prelimbic area of the eye, and an adjustment mechanism providing for mutual displacement of the optical units for setting them in accordance with an interpupillary distance of the patient, **characterized in that** the optical units are installed at an angle to each other providing coordination of reference directions with the directions of visual axes of the patient's eyes in a set angular interval during an observation with both eyes.

2. The device for laser therapy of claim 1, wherein the optical units are installed with the possibility of turning with respect to each other in an azimuthal plane.

3. The device for laser therapy in ophthalmology of claim 1, wherein in each optical unit the means for patient's sight fixation in a reference direction is installed with a possibility of displacement in a transversal plane with respect to the optical axis of the unit.

4. A device for laser therapy in ophthalmology, comprising two optical units each having one or several lasing emitters with forming optics for trans-scleral action by laser radiation upon one or several zones of a prelimbic area of the eye, and a source of light for patient's sight fixation specifying a reference direction, in which the forming optics provides for laser radiation hitting said zones of the prelimbic area of the eye, and an adjustment mechanism providing for mutual displacement of the optical units for setting them in accordance with an interpupillary distance of the patient, **characterized in that** in each optical unit the means for patient's sight fixation is displaced in a transversal plane with respect to the optical axis of the unit for a distance, providing for coordination of reference directions with the directions of visual axes of patient's eyes in the specified angular interval during an observation with both eyes.

5. The device for laser therapy in ophthalmology of claim 4, wherein in each optical unit one or several lasing emitters with the forming optics and the means for patient's sight fixation are installed with a possibility of coordinated displacement in a transversal plane with respect to the optical axis of the unit.

## Patentansprüche

1. Vorrichtung zur Lasertherapie in der Augenheilkunde, die Folgendes umfasst: zwei optische Einheiten, die jeweils eine oder mehrere lasernde Strahlungsquellen mit einer Formungsoptik zur transskleralen Wirkung durch Laserstrahlung auf eine oder mehrere Zonen eines prälimbischen Bereichs des Auges aufweisen, und eine Lichtquelle zur Fixierung des Sehvermögens des Patienten, die eine Bezugsrichtung spezifiziert, wobei die Formungsoptik eine Laserstrahlung bereitstellt, die auf die Zonen des prälimbischen Bereichs des Auges auftrifft, und einen Einstellmechanismus, der die gegenseitige Verschiebung der optischen Einheiten bereitstellt, um sie gemäß einer Pupillendistanz des Patienten einzustellen, **dadurch gekennzeichnet, dass** die optischen Einheiten in einem Winkel zueinander installiert sind, der die Koordination von Bezugsrichtungen mit den Richtungen der Sichtachsen der Augen des Patienten in einem eingestellten Winkelintervall während einer Beobachtung mit beiden Augen bereitstellt.

2. Vorrichtung zur Lasertherapie nach Anspruch 1, wobei die optischen Einheiten mit der Möglichkeit installiert sind, im Verhältnis zueinander in einer scheitelwinkligen Ebene zu drehen.

3. Vorrichtung zur Lasertherapie in der Augenheilkunde nach Anspruch 1, wobei bei jeder optischen Einheit das Mittel zur Fixierung des Sehvermögens des Patienten in einer Bezugsrichtung mit der Möglichkeit einer Verschiebung in einer transversalen Ebene im Verhältnis zu der optischen Achse der Einheit installiert ist.

4. Vorrichtung zur Lasertherapie in der Augenheilkunde, die Folgendes umfasst: zwei optische Einheiten, von denen jede eine oder mehrere lasernde Strahlungsquellen mit einer Formungsoptik zur transskleralen Wirkung durch Laserstrahlung auf eine oder mehrere Zonen eines prälimbischen Bereichs des Auges aufweisen, und eine Lichtquelle zur Fixierung des Sehvermögens des Patienten, die eine Bezugsrichtung spezifiziert, wobei die Formungsoptik eine Laserstrahlung bereitstellt, die auf die Zonen des prälimbischen Bereichs des Auges auftrifft, und einen Einstellmechanismus, der die gegenseitige Verschiebung der optischen Einheiten bereitstellt, um sie gemäß einer Pupillendistanz des Patienten einzustellen, **dadurch gekennzeichnet, dass** bei jeder optischen Einheit das Mittel zur Fixierung des Sehvermögens des Patienten in einer transversalen Ebene im Verhältnis zu der optischen Achse der Einheit um einen Abstand verschoben ist, wodurch eine Koordination von Bezugsrichtungen mit den Richtungen der Sichtachsen der Augen des Patienten in dem spezifizierten Winkelintervall während einer Beobachtung mit beiden Augen bereitgestellt wird.

5. Vorrichtung zur Lasertherapie in der Augenheilkunde nach Anspruch 4, wobei bei jeder optischen Einheit eine oder mehrere lasernde Strahlungsquellen mit der Formungsoptik und dem Mittel zur Fixierung des Sehvermögens des Patienten mit der Möglichkeit zu einer koordinierten Verschiebung in einer transversalen Ebene im Verhältnis zu der optischen Achse der Einheit installiert sind.

## Revendications

1. Dispositif de thérapie laser en ophtalmologie, comprenant deux unités optiques ayant chacune un ou plusieurs émetteurs laser avec une optique formatrice pour l'action trans-sclérale par rayonnement laser sur une ou plusieurs zones d'une région prélimbique de l'oeil, et une source de lumière pour la fixation du regard du patient spécifiquement dans une direction de référence, dans lequel l'optique formatrice permet au rayonnement laser de frapper lesdites zones de la région prélimbique de l'oeil, et un mécanisme d'ajustement qui permet le déplacement mutuel des unités optiques pour les placer conformément à une distance interpupillaire du patient, **caractérisé en ce que** les unités optiques sont installées à un angle l'une par rapport à l'autre permettant la coordination des directions de référence avec les directions des axes visuels des yeux du patient dans un intervalle angulaire fixe pendant une observation avec les deux yeux.

2. Dispositif de thérapie laser selon la revendication 1, dans lequel les unités optiques sont installées avec la possibilité de tourner l'une par rapport à l'autre dans un plan azimutal.

3. Dispositif de thérapie laser en ophtalmologie selon la revendication 1, dans lequel dans chaque unité optique le moyen de fixation du regard du patient dans une direction de référence est installé avec une possibilité de déplacement dans un plan transversal par rapport à l'axe optique de l'unité.

4. Dispositif de thérapie laser en ophtalmologie, comprenant deux unités optiques ayant chacune un ou plusieurs émetteurs laser avec des optiques formatrices pour l'action trans-sclérale par rayonnement laser sur une ou plusieurs zones d'une région prélimbique de l'oeil, et une source de lumière pour la fixation du regard du patient spécifiquement dans une direction de référence, dans lequel l'optique formatrice permet au rayonnement laser de frapper lesdites zones de la région prélimbique de l'oeil, et un mécanisme d'ajustement qui permet le déplacement mutuel des unités optiques pour les placer conformément à une distance interpupillaire du patient, **caractérisé en ce que** dans chaque unité optique le moyen de fixation du regard du patient est déplacé dans un plan transversal par rapport à l'axe optique de l'unité sur une distance, permettant la coordination des directions de référence avec les directions des axes visuels des yeux du patient dans l'intervalle angulaire spécifié pendant une observation avec les deux yeux.

5. Dispositif de thérapie laser en ophtalmologie selon la revendication 4, dans lequel dans chaque unité optique un ou plusieurs émetteurs laser avec les optiques formatrices et le moyen de fixation du regard du patient sont installés avec une possibilité de déplacement coordonné dans un plan transversal par rapport à l'axe optique de l'unité.
